# EUROPÄISCHE PATENTSCHRIFT

(11) **EP 4 134 002 B1**
(45) Veröffentlichungstag und Bekanntmachung des Hinweises auf die Patenterteilung: **05.03.2025**
(21) Anmeldenummer: 22185263.5
(22) Anmeldetag: 15.07.2022
(51) Int. Cl.: A61B 5/12, A61B 5/00, H04R 25/00

(54) **VERFAHREN UND HÖRSYSTEM ZUM ERSTELLEN EINES AUDIOGRAMMS EINER TESTPERSON MITTELS EINES HÖRINSTRUMENTS**
METHOD AND HEARING SYSTEM FOR CREATING AN AUDIOGRAM OF A SUBJECT BY MEANS OF A HEARING AID
PROCÉDÉ ET SYSTÈME DE GÉNÉRATION D'UN AUDIOGRAMME D'UNE PERSONNE SOUMISE À L'ESSAI AU MOYEN D'UN INSTRUMENT AUDITIF

(30) Priorität: 10.08.2021 DE 102021208735
(43) Veröffentlichungstag der Anmeldung: 15.02.2023
(73) Patentinhaber: Sivantos Pte. Ltd., Singapore 539775 (SG)
(72) Erfinder: GÖKAY, Umut, 55116 Mainz (DE)
(74) Vertreter: FDST Patentanwälte

(56) Entgegenhaltungen:
- WO-A1-2020/127939
- CH-A5- 678 692
- US-A1- 2018 021 176
- US-A1- 2018 063 618
- US-A1- 2021 076 146

## Beschreibung

Die Erfindung betrifft ein Verfahren nach Anspruch 1 zum Erstellen eines Audiogramms einer Testperson mittels eines Hörinstruments, wobei durch einen elektroakustischen Ausgangswandler des Hörinstruments ein Testschall erzeugt und in einen durch das Hörinstrument wenigstens teilweise verschlossenen Gehörgang der Testperson ausgegeben wird, und wobei anhand einer Reaktion der Testperson auf den Testschall eine Hörschwelle der Testperson bei wenigstens einer Testfrequenz ermittelt wird. Die Erfindung betrifft weiter ein Hörsystem nach Anspruch 6, mittels dessen sich eine Hörschwelle einer Testperson bei wenigstens einer Testfrequenz ermitteln lässt. Unter einem Hörinstrument ist generell jedwedes Gerät umfasst, welches dazu vorgesehen und eingerichtet ist, aus einem elektrischen Ausgangssignal mittels eines elektroakustischen Ausgangswandlers einen entsprechenden Ausgangsschall zu erzeugen, und diesen einem Gehör eines Benutzers zuzuführen. Als ein solcher Ausgangswandler kann hierbei insbesondere ein Lautsprecher verwendet werden, jedoch können insbesondere auch thermoakustische Wandler oder Knochenleithöherer verwendet werden. Ein Hörinstrument kann dabei einerseits lediglich auf die Erzeugung des Ausgangsschalls anhand von Audiodaten ausgelegt sein, also beispielsweise in der Form eines drahtlosen, insbesondere Ohrstöpselförmigen Kopfhörers. In diesem Fall wird ein Ausgangsschall anhand von Audiodaten erzeugt, welche beispielsweise durch Musik gegeben sein können, und welche vorab gespeichert wurden, oder auch über eine entsprechende Antenne zum Hörinstrument übertragen werden (per Stream).

Ein Hörinstrument kann aber auch als ein Hörgerät vorliegen, welches dazu eingerichtet ist, eine Hörschwäche eines Benutzers zu korrigieren bzw. wenigstens teilweise auszugleichen, indem beispielsweise mittels wenigstens eines elektroakustischen Eingangswandlers ein Umgebungsschall in ein entsprechendes elektrisches Eingangssignal umgewandelt wird, welches im Hörgerät gemäß der audiologischen Anforderungen des Benutzers verarbeitet und dabei insbesondere frequenzbandweise verstärkt wird, sodass das verarbeitete Eingangssignal über den elektroakustischen Ausgangswandler dem Gehör des Benutzers als Ausgangsschall zugeführt wird.

Insbesondere für die besagte Korrektur einer Hörschwäche des Benutzers im Fall eines Hörgerätes (im "engeren Sinn") wird meist ein Audiogramm des Benutzers erstellt. Dieses gibt für einzelne Testfrequenzen, welch bevorzugt das gesamte akustische Spektrum menschlichen Hörempfindens abdecken, die jeweilige Hörschwelle des Benutzers bei der betreffenden Frequenz an, sodass das Hörvermögen des Benutzers insbesondere mit dem Hörvermögen von normal hörenden Personen verglichen werden kann, um hieraus Schlüsse für die o.g. individualisierte Signalverarbeitung im Hörgerät zu gewinnen. Aber auch für eine Anpassung eines Hörinstruments (im "weiteren Sinn") an einen Benutzer zur Verbesserung des Klangempfindens, also z.B. für eine bessere akustische Performance in bestimmten Frequenzbereichen, kann das Erstellen eines Audiogramms von Vorteil sein.

Die Messung der Hörschwelle bei den einzelnen Testfrequenzen wird dabei meist bei einem Audiologen oder niedergelassenen Akustiker durchgeführt, wobei oftmals eine Testsonde mit einem Schallerzeuger in einen Gehörgang des Benutzers eingeführt wird. Einzelne Testtöne bei den jeweiligen Testfrequenzen werden jeweils mit stetig zu- oder abnehmendem Schallpegel erzeugt, sodass der Benutzer zu erkennen geben kann, ab wann er bei einer Testfrequenz den Testton hör bzw. nicht mehr hört. Ein Nachteil ist hierbei, dass derartige Messungen des Audiogramms bei dem bzw. durch den Audiologen/Akustiker durchzuführen sind.

Die CH 678 692 A5 nennt ein Verfahren zum Messen der individuellen akustischen Verhältnisse in einem Ohr. Hierbei wird der Ohrkanal durch eine passend geformte Ohrmulde für ein zum Einsatz vorgesehenes Im-Ohr-Hörgerät verschlossen, und die Messungen über einen in die Ohrmulde eingebauten Hörer und ein ebenfalls eingebautes Messmikrophon im abgeschlossenen Schallfeld direkt vor dem Trommelfell durchgeführt. Hörer und Mikrophon können als separate Bauteile ausgebildet sein, oder es können Mittel vorgesehen sein, welche den Hörer abwechslungsweise als Hörer bzw. als Mikrophon arbeiten lassen.

Die US 2021 / 0 076 146 A1 nennt ein Verfahren zum Betrieb eines Hörgerätes, das eine aktive Rauschunterdrückung zur Unterdrückung von Rauschsignalen mit einer oder mehreren Frequenzkomponenten durchführt. Es wird ein Audiogramm bereitgestellt, das eine Hörschwelle eines Benutzers des Hörgeräts als Funktion der Frequenz angibt, wobei unter Verwendung des Audiogramms bestimmt wird, welche Frequenzkomponenten der Rauschsignalen für den Benutzer hörbar sind und welche nicht hörbar sind. Die Rauschunterdrückung wird selektiv betrieben, indem hörbare Frequenzanteile der Rauschsignale unterdrückt werden und unhörbare Frequenzanteile der Rauschsignale nicht unterdrückt werden.

In der US 2018 / 0 063 618 A1 ist ein Ohrstück für ein Hörinstrument genannt, welches für die Durchführung audiometrischer Tests eingerichtet ist. Das Ohrstück umfasst ein Gehäuse, ein intelligentes Steuersystem, das innerhalb des Gehäuses angeordnet ist, mindestens einen Wandler, der operativ mit der intelligenten Steuerung verbunden ist, und mindestens einen Lautsprecher, der operativ mit der intelligenten Steuerung verbunden ist. Das Ohrstück ist dazu eingerichtet, dass es audiometrische Tests eines Benutzers durchführt, indem es Töne an dem mindestens einen Wandler wiedergibt und Benutzerrückmeldungen zu den Tönen empfängt, um audiometrische Testdaten zu liefern.

Die WO 2020 / 127 939 A1 offenbart ein Verfahren für einen lärm-kompensierten Hörtest. Das Verfahren zur Lärmkompensation eines Hörtests, der in einer unkontrollierten Umgebung durchgeführt wird, wird durchgeführt, indem ein oder mehrere Testtöne bei einer Anzahl von Testtonfrequenzen erzeugt werden, wobei die Testtöne einem Benutzer zugeführt werden, von diesem dann Benutzereingaben hinsichtlich der Wahrnehmung der Testtöne eingegeben werden, so dass ein Testergebnis bereitgestellt werden kann, das Intensitätspegel des Hörens für die Anzahl von Testtonfrequenzen umfasst, und ein Hörprofil auf der Grundlage des Testergebnisses erzeugt wird. Das Verfahren umfasst weiter das Auswerten eines von einem Mikrofon empfangenen Geräuschpegels, das Bestimmen eines Geräuschwahrnehmungsmodells auf der Grundlage des Geräuschpegels und das Kompensieren des Testergebnisses der Testtöne unter Verwendung des Geräuschwahrnehmungsmodells.

Der Erfindung liegt daher die Aufgabe zugrunde, ein Verfahren anzugeben, durch welches sich mittels eines Hörinstruments in möglichst einfacher und dennoch verlässlicher Weise sowie möglichst situationsunabhängig ein Audiogramm eines Trägers des Hörinstruments erstellen lässt. Der Erfindung liegt weiter die Aufgabe zugrunde, ein Hörinstrument anzugeben, welches zum möglichst einfachen und verlässlichen Erstellen eines Audiogramms eines Trägers eingerichtet ist.

Die erstgenannte Aufgabe wird erfindungsgemäß gelöst durch ein Verfahren nach Anspruch 1 zum Erstellen eines Audiogramms einer Testperson mittels eines Hörinstruments, wobei durch einen ersten elektroakustischen Eingangswandler des Hörinstruments ein Schallsignal in einem durch das Hörinstrument wenigstens teilweise verschlossenen Gehörgang der Testperson aufgenommen wird, und hieraus ein erstes Eingangssignal erzeugt wird, wobei durch einen elektroakustischen Ausgangswandler des Hörinstruments ein Testschall erzeugt und in den durch das Hörinstrument wenigstens teilweise verschlossenen Gehörgang der Testperson ausgegeben wird, und wobei anhand einer Reaktion der Testperson auf den Testschall und mittels des ersten Eingangssignals eine Hörschwelle der Testperson bei wenigstens einer Testfrequenz ermittelt wird. Vorteilhafte und teils für sich gesehen erfinderische Ausgestaltungen sind Gegenstand der Unteransprüche und der nachfolgenden Beschreibung.

Die zweitgenannte Aufgabe wird erfindungsgemäß gelöst durch ein Hörsystem nach Anspruch 6 mit einem Hörinstrument, umfassend einen ersten elektroakustischen Eingangswandler, welcher dazu eingerichtet ist, bei einem bestimmungsgemäßen Tragen des Hörinstruments durch eine Testperson ein Schallsignal in einem Gehörgang der Testperson aufzunehmen, und daraus ein erstes Eingangssignal zu erzeugen, einen elektroakustischen Ausgangswandler, welcher dazu eingerichtet ist einen Testschall zu erzeugen und bei einem bestimmungsgemäßen Tragen des Hörinstruments in den Gehörgang der Testperson auszugeben, und eine Steuereinrichtung, welche dazu eingerichtet ist, eine Reaktion der Testperson auf den Testschall zu registrieren und anhand besagter Reaktion der Testperson auf den Testschall sowie mittels des ersten Eingangssignals eine Hörschwelle der Testperson bei wenigstens einer Testfrequenz zu ermitteln.

Das erfindungsgemäße Hörsystem teilt die Vorzüge des erfindungsgemäßen Verfahrens. Die für das Verfahren und für seine Weiterbildungen angegebenen Vorteile können sinngemäß auf das Hörsystem übertragen werden.

Die Steuereinrichtung umfasst bevorzugt ein wenigsten einen Signalprozessor und einem durch den Signalprozessor adressierbaren Arbeitsspeicher, welche zur Durchführung der genannten Signalverarbeitungsschritte eingerichtet sind. Das Hörsystem kann dabei einerseits durch das Hörinstrument gegeben sein, oder auch eine mit dem Hörinstrument datentechnisch verbindbare Hilfsvorrichtung aufweisen, z.B. ein Smartphon oder einen Tablet-PC, wobei die Steuereinrichtung in letzterem Fall durch eine Steuereinheit im Hörinstrument und/oder eine Steuereinheit auf der Hilfsvorrichtung gegeben sein kann.

Unter einem elektroakustischen Eingangswandler ist hierbei insbesondere jedweder Wandler umfasst, welcher dazu eingerichtet ist, aus einem Umgebungsschall ein elektrisches Audiosignal zu erzeugen, sodass durch den Umgebungsschall hervorgerufene Luftbewegungen und Luftdruckschwankungen am Ort des Wandlers durch entsprechende Oszillationen einer elektrischen Größe, insbesondere einer Spannung im erzeugten Audiosignal wiedergegeben werden. Insbesondere kann der elektroakustische Eingangswandler durch ein Mikrofon gegeben sein. Entsprechend ist unter einem elektroakustischer Ausgangswandler jedweder Wandler umfasst, welcher dazu eingerichtet ist, aus einem elektrischen Ausgangssignal einen Ausgangsschall zu erzeugen, also insbesondere ein einen Lautsprecher (wie etwa ein Balanced Metal Case Receiver), aber z.B. auch ein thermoakustischer Wandler.

Unter einer Aufnahme eines Schallsignals in einem durch das Hörinstrument wenigstens teilweise verschlossenen Gehörgang der Testperson durch den ersten elektroakustischen Eingangswandler des Hörinstruments ist insbesondere umfasst, dass das Hörinstrument durch die Testperson, insbesondere in einer de bestimmungsgemäßen Gebrauch des Hörinstruments entsprechenden Weise, an einem Ohr getragen wird, und hierbei der Gehörgang des betreffenden Ohres zumindest durch einen Teil des Hörinstruments wenigstens teilweise nach außen hin verschlossen wird. Insbesondere kann hierbei ein Teil des Hörinstruments in der Concha durch Druck fest anliegen und/oder ein Teil in den Gehörgang hineinragen und durch Druck dort fest anliegen.

Das Hörinstrument ist dabei bevorzugt so ausgestaltet, dass der erste elektroakustische Eingangswandler beim bestimmungsgemäßen Tragen des Hörinstruments am Ohr in den betreffenden Gehörgang ausgerichtet ist. Das durch den ersten elektroakustischen Eingangswandler aufgenommene Schallsignal ist insbesondere durch denjenigen Schall gegeben, welcher sich zwischen dem Trommelfell und dem Hörinstrument ausbreitet, und kann einen hohen Anteil an Körperschall enthalten, welcher infolge des teilweisen Verschlusses des Gehörgangs durch das Hörinstrument nicht in die Umgebung entweichen kann (sog. Okklusionseffekt). Das erste Eingangssignal enthält daher insbesondere Signalbeträge des besagten Körperschalls.

Der Testschall weist bei der wenigstens einen Testfrequenz bevorzugt einen definierten ersten Signalbeitrag, also insbesondere einen definierten Schallpegel im Bereich der ersten Testfrequenz auf. Insbesondere kann dieser definierte erste Signalbeitrag bzw. definierte Schallpegel im Bereich der ersten Testfrequenz variiert werden, also bspw. von einem aller Wahrscheinlichkeit nach für jedwede Testperson unhörbaren Schallpegel aus stetig erhöht werden, oder von einem aller Wahrscheinlichkeit nach für jedwede Testperson hörbaren Schallpegel stetig erhöht werden. Das Hörinstrument ist dabei bevorzugt so ausgestaltet, dass der elektroakustische Ausgangswandler beim bestimmungsgemäßen Tragen des Hörinstruments am Ohr in den betreffenden Gehörgang ausgerichtet ist.

Die Reaktion der Testperson auf den Testschall kann hierbei insbesondere als ein Sprachsignal durch einen zweiten elektroakustischen Eingangswandler des Hörinstruments erfasst werden, welcher in die freie Umgebung des Hörinstruments ausgerichtet ist, und dazu eingerichtet ist, aus dem Umgebungsschall des Hörinstrumentes ein zweites Eingangssignal zu erzeugen. Das zweite Eingangssignal kann dann durch eine Steuereinrichtung des Hörinstruments entsprechend auf Sprachbefehle hin analysiert werden, welche eine Reaktion auf den Testschall beinhalten. Die Reaktion kann jedoch insbesondere auch mittels einer Hilfsvorrichtung erfasst werden, welche von der Testperson entsprechend angesteuert wird, z.B. mittels einer entsprechenden App zur Erfassung der Reaktion über eine Bildschirmeingabe der Testperson. Die Reaktion kann jedoch auch als eine Bewegung der Testperson mittels eines entsprechen eingerichteten Sensors des Hörinstruments erfasst werden, also z.B. als ein Kopfnicken oder Kopfschütteln zum Signalisieren, dass der Testschall gehört bzw. nicht (mehr) gehört wird, bspw. mittels eines Bewegungs- und/oder Beschleunigungssensors und/oder eines Gyroskops.

Die Hörschwelle bei der wenigstens einen Testfrequenz kann dabei insbesondere dadurch ermittelt werden, dass in oben beschriebener Weise der Testschall mit variablem, bevorzugt stetig ansteigendem oder abnehmendem Schallpegel im Bereich der ersten Testfrequenz in den Gehörgang der Testperson ausgegeben wird, und die Testperson über die Reaktion zu erkennen gibt, ab wann der Testschall gehört wird (ansteigend) bzw. wann der Testschall nicht mehr zu hören ist (abnehmend).

Hierbei kann das erste Eingangssignal einerseits dahingehend für die Bestimmung der Hörschwelle herangezogen werden, dass anhand des ersten Eingangssignals vor einer Emission des Testschalls eine Art "Rauschhintergrund" im Gehörgang ermittelt wird, und dieser Rauschhintergrund zur Bestimmung des Schallpegels bei der wenigsten einen Testfrequenz mit berücksichtigt wird (der andere wesentliche Signalbeitrag bei der Testfrequenz stammt vom Testschall, dessen definierter Schallpegel mit dem Rauschhintergrund für den endgültigen Schallpegel bei der Testfrequenz zu verrechnen ist). Andererseits wird anhand des ersten Eingangssignals der definierte erste Signalbeitrag der Testfrequenz im Testschall auch adaptiv und insbesondere während der Ausgabe des Testschalls angepasst, wofür insbesondere eine Filterung des ersten Eingangssignals hinsichtlich des ersten Signalbeitrags bei der wenigstens einen Testfrequenz erfolgen kann. Überdies wird erfindungsgemäß anhand des ersten Eingangssignals eine aktive Okklusionsunterdrückung durchgeführt. Hierzu wird anhand des ersten Eingangssignals ein Korrektursignal für die Okklusionsunterdrückung erzeugt, welches durch den elektroakustischen Ausgangswandler in einen Korrekturschall umgewandelt wird. Das Korrektursignal kann dabei zur Ausgabe dem für die Erzeugung des Testschalls vorgesehenen Signal überlagert werden.

Das Audiogramm wird für die wenigstens eine Testfrequenz anhand und bevorzugt unmittelbar durch die so ermittelte Hörschwelle bestimmt. Das Audiogramm kann nun vollständig in der beschriebenen Weise erstellt werden, indem eine Mehrzahl an Testfrequenzen vorgegeben wird, und sukzessive ein entsprechender Testschall mit einem definierten Signalbeitrag bei der jeweiligen Testfrequenz erzeugt und in den Gehörgang der Testperson ausgegeben wird, und für jede der Testfrequenzen die Hörschwelle anhand der jeweiligen Reaktion der Testperson auf den betreffenden Testschall sowie anhand des ersten Eingangssignals ermittelt wird. Bevorzugt decken dabei die Testfrequenzen das menschliche Hörsektrum vollständig ab, und ermöglichen zudem eine möglichst vorteilhafte Frequenzauflösung des Hörspektrums (also z.B. eine im Wesentlichen gleichbleibende Anzahl an Testfrequenzen für jedwede beliebig ausgewählte Oktave).

Die für die wenigstens eine Testfrequenz wie beschrieben ermittelte Hörschwelle kann jedoch auch zum Ersatz der entsprechenden Daten in einem bereits bestehenden bzw. vorgegebenen Audiogramm, oder als eine Ergänzung zu einem solchen verwendet werden.

Gerade für ein Hörinstrument, welches für den bestimmungsgemäßen Betrieb so an einen Ohr zu tragen ist, dass dabei der betreffende Gehörgang in erheblichem Maße verschlossen wird, kann eine Messung einer Hörschwelle mittels des Hörinstruments durch den besagten Verschluss des Gehörgangs infolge von Okklusionseffekten erschwert oder auch verfälscht werden. Das vorgeschlagene Verfahren löst diese Problematik, indem Geräusche im Gehörgang, welche nicht zum Testschall und insbesondere zum ersten Signalbeitrag des Testschalls bei der wenigstens einen Testfrequenz gehören, aktiv abgeschwächt werden können, oder ihr Beitrag zum Gesamtschallpegel im Gehörgang im Bereich der wenigstens einen Testfrequenz berücksichtigt werden kann. Hierdurch lässt sich infolge der Vermeidung der o.g. Verfälschungen die Hörschwelle auch durch das Hörinstrument ermitteln. Dadurch kann es der Testperson erspart werden, für eine Neuerstellung eines Audiogramms oder auch nur eine Anpassung bzw. Korrektur des Audiogramms einen Audiologen oder niedergelassenen Akustiker aufsuchen zu müssen.

Bevorzugt weist der Testschall einen definierten ersten Signalbeitrag bei der wenigsten einen Testfrequenz auf, wobei anhand des ersten Eingangssignals ein zweiter Signalbeitrag des Schallsignals im Gehörgang bei der wenigstens einen Testfrequenz ermittelt wird, und wobei für ein Ermitteln der Hörschwelle bei der wenigstens einen Testperson wenigstens der zweite Signalbeitrag berücksichtigt wird. Dies kann insbesondere dadurch erfolgen, dass der zweite Signalbeitrag den gesamten Schall im Gehörgang bei der wenigstens einen Testfrequenz erfasst, welcher sich zusammensetzt aus dem definierten ersten Signalbeitrag des Testschalls, und einem Schallbeitrag, welcher weitgehend durch Körperschall bedingt ist und ggf. noch Umgebungsschall enthält, welcher am meist unvollständigen Verschluss des Gehörgangs durch das Hörinstrument vorbei in den Gehörgang gelangt. A priori ist im Hörinstrument nur der durch dieses selbst erzeugte erste Signalbeitrag im Testschall bekannt, wodurch eine Reaktion auf die Summe des Testschalls und der anderen genannten Beiträge ggf. zu einer unrichtigen, insbesondere zu niedrigen Hörschwelle führen kann. Durch die Berücksichtigung des zweiten Signalbeitrags kann dies korrigiert werden, indem der erste Signalbeitrag entsprechend angepasst wird.

Der zweie Signalbeitrag kann jedoch - insbesondere nach einer Filterung des ersten Eingangssignals bzgl. des ersten Signalbeitrags - auch lediglich die durch Körper- und ggf. Umgebungsschall bedingten Beiträge aufweisen, sodass für die Bestimmung des Schalpegels im Gehörgang (und entsprechend für die korrekte Hörschwelle) in diesem Fall der erste und der zweite Signalbeitrag heranzuziehen sind.

Erfindungsgemäß wird anhand des ersten Eingangssignals ein Korrektursignal für eine aktive Okklusionsunterdrückung erzeugt, wobei durch den elektroakustischen Ausgangswandler des Hörinstruments anhand des Korrektursignals zur Kompensation eines Körperschalls im Schallsignal des Gehörgangs ein Korrekturschall erzeugt und in den (durch das Hörinstrument wenigstens teilweise verschlossenen) Gehörgang der Testperson ausgegeben wird. Eine aktive Okklusionsunterdrückung ermittelt Geräusche in einem weitgehen verschlossenen Gehörgang, welche auf Körperschall basieren, und erzeugt dann im Gehörgang einen Kompensationsschall, welcher den Körperschall möglichst kompensiert. Vorliegend können die auf Körperschall basierenden Signalbeiträge im Gehörgang anhand des ersten Eingangssignals ermittelt werden, welches dafür bevorzugt um den durch den Testschall bedingten definierten ersten Signalbeitrag mittels entsprechender Filterung zu bereinigen ist. Für die so ermittelten, residualen Signalbeiträge wird nun zur Kompensation ein entsprechendes, bevorzugt gegenphasiges Korrektursignal erzeugt, welches der elektroakustische Ausgangswandler des Hörinstruments in einen Korrekturschall umwandelt, der in den Gehörgang ausgegeben wird.

Die so beschriebene Kompensation von Körperschall infolge von Okklusionseffekten, welche bevorzugt breitbandig erfolgt, senkt dabei den Schallpegel im Gehörgang, wodurch der Testperson einerseits eine bessere Konzentration auf den Testschall und insbesondere dessen ersten Signalbeitrag bei der Testfrequenz ermöglicht wird, und andererseits Maskierungseffekte durch Schallereignisse abseits der Testfrequenz verhindert werden können, welche gerade bei geringen Schalpegeln des Testschalls (wie sie ja zum Ermitteln der Hörschwelle nahezu zwangsläufig auftreten) relevant werden. Eine etwaige zusätzlich erfolgende Kompensation von durch Umgebungsschall bedingten Geräuschen kann sich dabei weiter positiv auf das Ermitteln der Hörschwelle auswirken.

Vorteilhafterweise wird dabei der Korrekturschall als ein breitbandiges Schallsignal erzeugt und ausgegeben, wobei als der zweite Signalbeitrag anhand des ersten Eingangssignals ein von der aktiven Okklusionsunterdrückung nicht bereinigtes, residuales Rauschsignal ermittelt wird. Dieser zweite Signalbeitrag wird insbesondere für die Erzeugung des Testschalls berücksichtigt, indem entsprechend des zweiten Signalbeitrags der erste Signalbeitrag des Testschalls bei der Testfrequenz eingestellt wird, um im Bereich der Testfrequenz den gewünschten Schallpegel im Gehörgang zu erreichen.

Erfindungsgemäß wird durch einen zweiten elektroakustischen Eingangswandler des Hörinstruments ein Umgebungsschall des Hörinstrumentes aufgenommen wird, und hieraus ein zweites Eingangssignal erzeugt, wobei anhand des zweiten Eingangssignals durch den elektroakustischen Ausgangswandler des Hörinstruments zusätzlich zur Ausgabe des Testschalls eine aktive Rauschunterdrückung ("active noise cancelling", ANC) durchgeführt wird. Eine ANC ermittelt Geräusche im Umgebungsschall, und erzeugt dann im Gehörgang einen Kompensationsschall, welcher die aus der Umgebung in den Gehörgang gelangenden möglichst kompensiert. Im Fall, dass das Hörinstrument als ein Hörgerät gegeben ist, weist dieses für den Normalbetrieb bereits wenigstens einen elektroakustischen Eingangswandler zur Aufnahme von Umgebungsschall auf. Aber auch Hörinstrumente ohne spezifische Korrektur einer Hörschwäche wesen oft einen solchen Eingangswandler zur Aufnahme von Umgebungsschall auf, um z.B. Sprachbefehle des Benutzers empfangen zu können, oder auch für Telefonie-Funktionen. Das für die ANC erzeugte Korrektursignal kann dabei zur Ausgabe dem für die Erzeugung des Testschalls vorgesehenen Signal überlagert werden. Durch die ANC ist die Testperson bei der Ermittlung der Hörschwelle in geringerem Maße den Umgebungsgeräuschen ausgesetzt, sodass auch hier Verfälschungen und auch Maskierungseffekte weiter unterbunden werden.

Zweckmäßigerweise wird dabei die aktive Rauschunterdrückung auf einen Frequenzbereich um die wenigstens eine Testfrequenz konzentriert. Üblicherweise weist eine ANC einen bestimmten Arbeitsbereich auf, in welchem ein Rauschen besonders effizient unterdrückt wird. Hierbei lässt sich ein schmalbandiger Frequenzbereich von z.B. 50 Hz oder 100 Hz oder 250 Hz um die Testfrequenz besser auf eine vollständige Rauschunterdrückung hin optimieren, während etwa eine breitbandigere ANC vielleicht insgesamt eine stärkere Reduktion der gesamten Rauschenergie erreicht, wobei jedoch gerade im Umfeld der Testfrequenz ggf. ein größeres Restrauschen verbleiben kann, welches durch die ANC nicht beseitigt wird. Somit wird hier die Bandbreite der ANC zum Zweck einer möglichst vollständigen Rauschunterdrückung bei der Testfrequenz und vorzugsweise in ihrer unmittelbaren Umgebung verringert. Eine ANC liefert die besten Ergebnisse bei stark prädizierbaren Signalen, welche einen hohen tonalen Anteil und somit eine klare Lokalisierung im Frequenzspektrum aufweisen. Für das Ermitteln der Hörschwelle bedeutet dies, dass die ANC bevorzugt dann anzuwenden ist, wenn in der Umgebung der Testperson ein stark tonales Rauschsignal im Bereich der Testfrequenz auftritt.

Als vorteilhaft erweist es sich dabei, wenn die aktive Rauschunterdrückung auf einen Frequenzbereich um die wenigstens eine Testfrequenz konzentriert wird, und die aktive Okklusionsunterdrückung durch das Korrektursignal breitbandig erfolgt. Das Kompensationssignal der aktiven Okklusionsunterdrückung wird, anders als im Fall der ANC, bevorzugt in einer Rückkopplungsschleife erzeugt, wodurch sich andere Möglichkeiten bei der Kompensation der Geräusche ergeben. Da Okklusionseffekte oftmals mit größerer spektraler Breite auftreten, kann es von Vorteil sein, die Kompensation mittels des Korrekturschalls nicht auf bestimmte Frequenzbereiche zu beschränken. Insbesondere können die Korrektursignale der ANC und der aktiven Okklusionsunterdrückung dabei zur Ausgabe dem für die Erzeugung des Testschalls vorgesehenen Signal überlagert werden.

In einer vorteilhaften Ausgestaltung ist das Hörinstrument als ein Hörgerät ausgestaltet, und umfasst weiter einen zweiten elektroakustischen Eingangswandler, welcher dazu eingerichtet ist, einen Umgebungsschall des Hörgerätes aufzunehmen, und hieraus ein zweites Eingangssignal zu erzeugen, wobei die Steuereinrichtung weiter dazu eingerichtet ist, anhand des zweiten Eingangssignals ein Ausgangssignal zu erzeugen und dem elektroakustischen Ausgangswandler zur Umwandlung in ein Ausgangsschallsignal zuzuführen. Gerade für Hörgeräte ist das Erstellen eines Audiogramms durch einen Benutzer und ohne audiologische Betreuung von Vorteil.

Zweckmäßigerweise ist die Steuereinrichtung weiter dazu eingerichtet, anhand des zweiten Eingangssignals mittels des elektroakustischen Ausgangswandlers zusätzlich zur Ausgabe des Testschalls eine aktive Rauschunterdrückung durchzuführen.

Nachfolgend wird ein Ausführungsbeispiel der Erfindung anhand von Zeichnungen näher erläutert. Hierbei zeigt:

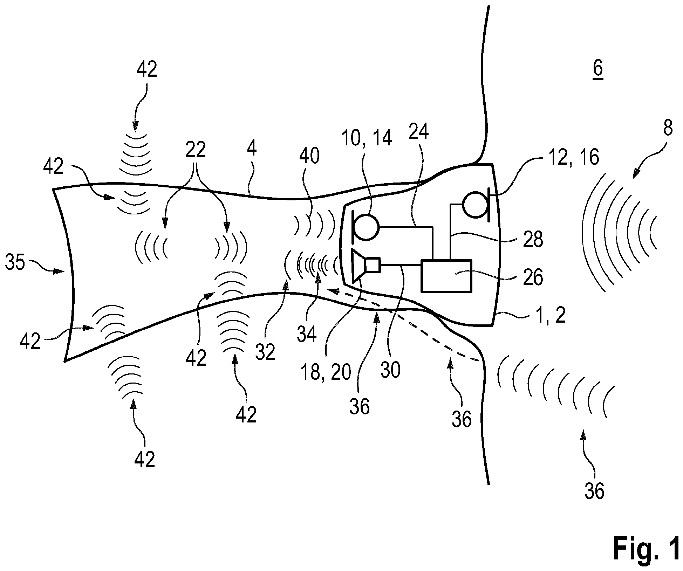

- Fig. 1: in einer Schnittdarstellung schematisch ein Hörinstrument, welches einen Gehörgang verschließt, und mittels dessen eine Hörschwelle eines Benutzers ermittelt werden kann.

In Figur 1 ist schematisch und nicht maßstabsgetreu in einer Schnittdarstellung ein Hörinstrument 1 gezeigt, welches vorliegend als ein Hörgerät 2 ausgestaltet ist. Das Hörgerät 2 ist ein einen Gehörgang 4 eingeführt, und sitzt dabei durch Druck auf die Haut am Gehörgang 4 in seiner Position fest. Hierdurch wird der Gehörgang 4 weitgehend gegenüber der Umgebung 6 verschlossen, so dass am Hörgerät 2 vorbei - über einen nicht vollständigen Verschluss des Gehörgangs 4 infolge eines nicht exakt im Gehörgang 4 anliegenden Sitzes des Hörgerätes 2 - sowie durch die dünnen Hautpartien am Eingang des Gehörgangs 4 noch ein geringer Anteil eines Umgebungsschalls 8 in den Gehörgang 4 gelangt.

Das Hörgerät 2 weist einen ersten elektroakustischen Eingangswandler 10 und einen zweiten elektroakustischen Eingangswandler 12 auf, welche vorliegend als ein erstes bzw. zweites Mikrofon 14 16 gegeben sind, sowie einen elektroakustischen Ausgangswandler 18, welcher als ein Lautsprecher 20 gegeben ist. Das erste Mikrofon 14 und der Lautsprecher 20 sind dabei bei bestimmungsgemäßen tragen des Hörgerätes 2 in den Gehörgang 4 gerichtet, das zweite Mikrofon 16 zur freien Umgebung 6 des Hörgerätes 2 hin.

Das erste Mikrofon ist dazu eingerichtet, ein Schallsignal 22 im Gehörgang 4 aufzunehmen und daraus ein erstes Eingangssignal 24 zu erzeugen, welches einer Steuereinrichtung 26 zugeführt wird. Das zweite Mikrofon 16 ist dazu eingerichtet, aus dem Umgebungsschall 8 ein zweites Eingangssignal 28 zu erzeugen, welches ebenfalls der Steuereinrichtung 28 zugeführt wird. Insbesondere kann das Hörgerät 2 noch einen weiteren, zur freien Umgebung 6 hin ausgerichteten elektroakustischen Eingangswandler (nicht dargestellt) aufweisen, welcher dazu eingerichtet ist, aus dem Umgebungsschall 8 ein weiteres Eingangssignal zu erzeugen.

Im Normalbetrieb des Hörgerätes 2 wird in der Steuereinrichtung 26 das zweite Eingangssignal (und ggf. besagtes weiteres Eingangssignal) frequenzbandspezifisch verarbeitet und insbesondere verstärkt, und hieraus ein Ausgangssignal 30 erzeugt, welches dem Lautsprecher 20 zugeführt wird. Hierzu kann die Steuereinrichtung 26 insbesondere einen Signalprozessor (nicht dargestellt) und einen vom Signalprozessor adressierbaren Arbeitsspeicher aufweisen. Der Lautsprecher 20 erzeugt aus dem Ausgangssignal 30 ein Ausgangsschallsignal (nicht dargestellt). Für die Erzeugung des Ausgangssignals 30 wird dabei durch die Steuereinrichtung 26 eine Hörschwäche eines Benutzers des Hörgerätes 2 berücksichtigt, indem u.a. eine frequenzbandspezifische Verstärkung gemäß der audiologischen Anforderungen des Benutzers zur Kompensation seiner Hörschwäche erfolgt. Hierfür wird im Hörgerät 2 ein Audiogramm als Information dieser audiologischen Anforderungen benötigt.

Üblicherweise wird ein derartiges Audiogramm, welches die Hörschwelle des Benutzers für einzelne Frequenzen angibt, bei einem Audiologen oder einem Akustiker erstellt, welchen der Benutzer hierfür aufsucht.

Das Hörgerät 2 ist jedoch durch einige nachfolgend beschriebene Besonderheiten dazu eingerichtet, dass ein Audiogramm auch mithilfe des Hörgerätes 2 erstellt werden kann, bzw. für einige Frequenzen ein bereits bestehendes (und bevorzugt in einem nicht-flüchtigen Speicher des Hörgerätes 2 insbesondere auch zur Nutzung im laufenden Betrieb hinterlegtes) Audiogramm aktualisiert werden kann.

Um mittels des Hörgerätes 2 eine Hörschwelle des Benutzers ermitteln zu können (und diese also für ein Audiogramm verwenden zu können), ist das Hörgerät 2 in vorteilhafter Weise dazu ausgestaltet, Störgeräusche zu unterdrücken, welche eine Messung einer Hörschwelle erschweren ("inconclusive result") oder auch verfälschen (fehlerhaftes Ergebnis) könnten, und welche üblicherweise in der spezifisch für derartige Messungen eingerichteten Umgebung bei einem Audiologen oder Akustiker nicht in nennenswertem Maß auftreten.

Durch den elektroakustischen Ausgangswandler 18 wird ein Testschall 32 ausgegeben, welcher durch entsprechende Beiträge im durch den elektroakustischen Ausgangswandler 18 gewandelten Ausgangssignal 30 erzeugt wird. Der Testschall 32 weist dabei einen definierten ersten Signalbeitrag 34 im Bereich einer Testfrequenz auf, für welche die Hörschwelle ermittelt werden soll. Der Testschall 32 mit dem ersten Signalbeitrag 34 propagiert dabei durch den Gehörgang 4 zum Trommelfell 36 des Benutzers, und wird durch den Benutzer abhängig vom Schallpegel des ersten Signalbeitrags 34 wahrgenommen. Für eine Messung der Hörschwelle bei der Testfrequenz wird dabei der erste Signalbeitrag 34 variiert (also z.B. von einem nicht wahrnehmbaren Startwert stetig gesteigert, oder von einem gut wahrnehmbaren Startwert stetig verringert), sodass der Benutzer eine Veränderung seiner Wahrnehmung (Testschall wird hörbar bzw. wird unhörbar) übermitteln kann.

Dies kann z.B. mittels einer entsprechenden Spracheingabe über den zweiten elektroakustischen Eingangswandler 12 erfolgen, wobei das zweite Eingangssignal 28 während der Ausgabe des Testschalls 32 auf eine derartige Spracheingabe hin zu analysieren ist. Es ist ebenso möglich, eine Benutzereingabe mittels eines Smartphones, eines Tablet-PCs (beide nicht dargestellt) o.ä. durchzuführen, welches bzw. welcher mit dem Hörgerät 2 bevorzugt drahtlos verbunden ist, wobei insbesondere eine entsprechend eingerichtete App für die Eingabe und auch für die Auswahl an Testfrequenzen o.ä. verwendet werden kann.

Damit die Messung Hörschwelle nicht durch externe Störgeräusche 36 im Umgebungsschall 8 erschwert oder verfälscht wird, die über den schematisch dargestellten Schallweg 38 in den Gehörgang 4 (und somit weiter zum Trommelfell 35 des Benutzers) propagieren, werden die externen Störgeräusche 36 über eine ANC kompensiert, indem anhand des vom zweiten elektroakustischen Eingangswandler 12 (welcher ja den Umgebungsschall 8 und somit die externen Störgeräusche 36 erfasst) erzeugten zweiten Eingangssignal 28 in der Steuereinrichtung 26 ein Kompensationssignal (nicht dargestellt) erzeugt wird, welches in das Ausgangssignal 30 eingeht. Das Kompensationssignal wird dabei in Phase und Amplitude derart erzeugt, dass die zugehörigen Signalbeiträge im durch den Lautsprecher 20 erzeugten Ausgangsschallsignal die Anteile der externen Störgeräusche 36, welche über den Schallweg 38 in den Gehörgang 4 propagiert sind, möglichst vollständig kompensieren.

Des Weiteren wird mittels des ersten elektroakustischen Eingangswandlers 10 bei der Testfrequenz des Testschalls 32 ein zweiter Signalbeitrag 40 im Schallsignal 22 im Gehörgang 4 ermittelt. Dies erfolgt mit der Zielsetzung, durch eine aktive Okklusionsunterdrückung einen in den Gehörgang 4 eindringenden Körperschall 42 zu unterdrücken. Der Anteil an Körperschall 4 im Schallsignal 22 kann durch das vom ersten elektroakustischen Eingangswandler 10 erzeugte erste Eingangssignal 24 erfasst werden, sodass in Abhängigkeit des erfassten Körperschalls 42 ein Korrektursignal (nicht dargestellt) erzeugt werden kann, welches in das Ausgangssignal 30 eingeht. Das entsprechend vom elektroakustischen Ausgangswandler 18 erzeugte Ausgangsschallsignal beinhaltet dann einen Korrekturschall, welcher den Körperschall 42 möglichst vollständig kompensiert. Hierbei kann eine Erfassung des Körperschalls 42 bevorzugt zeitlich getrennt von einer Ausgabe des Testschalls 32 erfolgen, sodass z.B. kurz vor einer solchen Ausgabe der zu kompensierende Körperschall 42 ermittelt wird, und eine entsprechende Kompensation mittels des Korrekturschalls während der Ausgabe des Testschalls erfolgt. Das vom elektroakustischen Ausgangswandler 18 erzeugte Ausgangsschallsignal kann somit den Testschall 32 sowie Beiträge der aktiven Okklusionsunterdrückung zur Kompensierung des Körperschalls 42 im Schallsignal 22 im Gehörgang 4 und Beiträge einer ANC zur Kompensierung der externen Störgeräusche 36 im Umgebungsschall 8 erfassen.

Alternativ dazu kann der Körperschall 42 auch während der Ausgabe des Testschalls 32 erfolgen (nicht dargestellt). In diesem Fall beinhaltet der zweite Signalbeitrag 40 des Schallsignals 22 im Gehörgang 4 auch den ersten Signalbeitrag 34 des Testschalls 32 bei der Testfrequenz. Der zweite Signalbeitrag 40 kann dann dazu verwendet werden, den ersten Signalbeitrag 34 im Testschall 32 so einzustellen, dass im Bereich der Testfrequenz als Summensignal aus Körperschall 42 und erstem Signalbeitrag 32 jeweils ein gewünschter Schallpegel am Trommelfell 35 auftrifft. Der zweite Signalbeitrag 34 dient hierbei somit einer direkten Korrektur des ersten Signalbeitrags 32 im Testschall 32.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung, wie durch die Patentansprüche definiert, zu verlassen.

### Bezugszeichenliste

- 1: Hörinstrument
- 2: Hörgerät
- 4: Gehörgang
- 6: freie Umgebung
- 8: Umgebungsschall
- 10: erster elektroakustischer Eingangswandler
- 12: zweiter elektroakustischer Eingangswandler
- 14: erstes Mikrofon
- 16: zweites Mikrofon
- 18: elektroakustischer Ausgangswandler
- 20: Lautsprecher
- 22: Schallsignal
- 24: erstes Eingangssignal
- 26: Steuereinrichtung
- 28: zweites Eingangssignal
- 30: Ausgangssignal
- 32: Testschall
- 34: erster Signalbeitrag
- 35: Trommelfell
- 36: externe Störgeräusche
- 38: Schallweg
- 40: zweiter Signalbeitrag
- 42: Körperschall

## Patentansprüche

1. Verfahren zum Erstellen eines Audiogramms einer Testperson mittels eines Hörinstruments (1),
wobei durch einen ersten elektroakustischen Eingangswandler (10) des Hörinstruments (1) ein Schallsignal (22) in einem durch das Hörinstrument (1) wenigstens teilweise verschlossenen Gehörgang (4) der Testperson aufgenommen wird, und hieraus ein erstes Eingangssignal (24) erzeugt wird,
wobei durch einen elektroakustischen Ausgangswandler (18) des Hörinstruments (1) ein Testschall (32) erzeugt und in den durch das Hörinstrument (1) wenigstens teilweise verschlossenen Gehörgang (4) der Testperson ausgegeben wird, und wobei
anhand einer Reaktion der Testperson auf den Testschall (32) und mittels des ersten Eingangssignals (24) eine Hörschwelle der Testperson bei wenigstens einer Testfrequenz ermittelt wird,
**dadurch gekennzeichnet dass**,
- anhand des ersten Eingangssignals (24) ein Korrektursignal für eine aktive Okklusionsunterdrückung erzeugt wird, und durch den elektroakustischen Ausgangswandler (18) des Hörinstruments (1) anhand des Korrektursignals zur Kompensation eines Körperschalls (42) im Schallsignal (22) des Gehörgangs (4) ein Korrekturschall erzeugt und in den durch das Hörinstrument (1) wenigstens teilweise verschlossenen Gehörgang (4) der Testperson ausgegeben wird, und/oder
- durch einen zweiten elektroakustischen Eingangswandler (12) des Hörinstruments (1) ein Umgebungsschall (8) des Hörinstrumentes (1) aufgenommen wird, und hieraus ein zweites Eingangssignal (28) erzeugt wird, und anhand des zweiten Eingangssignals (28) durch den elektroakustischen Ausgangswandler (18) des Hörinstruments (1) zusätzlich zur Ausgabe des Testschalls (32) eine aktive Rauschunterdrückung durchgeführt wird.

2. Verfahren nach Anspruch 1,
wobei der Testschall (32) einen definierten ersten Signalbeitrag (34) bei der wenigsten einen Testfrequenz aufweist,
wobei anhand des ersten Eingangssignals (24) ein zweiter Signalbeitrag (40) des Schallsignals (22) im Gehörgang (4) bei der wenigstens einen Testfrequenz ermittelt wird, und
wobei für ein Ermitteln der Hörschwelle bei der wenigstens einen Testperson wenigstens der zweite Signalbeitrag (40) berücksichtigt wird.

3. Verfahren nach Anspruch 1 oder Anspruch 2,
wobei der Korrekturschall als ein breitbandiges Schallsignal erzeugt und ausgegeben wird, und
wobei als der zweite Signalbeitrag (40) anhand des ersten Eingangssignals (24) ein von der aktiven Okklusionsunterdrückung nicht bereinigtes, residuales Rauschsignal ermittelt wird.

4. Verfahren nach einem der vorhergehenden Ansprüche, insofern eine aktive Rauschunterdrückung durchgeführt wird, wobei die aktive Rauschunterdrückung auf einen Frequenzbereich um die wenigstens eine Testfrequenz konzentriert wird.

5. Verfahren nach Anspruch 4,
wobei die aktive Rauschunterdrückung auf einen Frequenzbereich um die wenigstens eine Testfrequenz konzentriert wird, und
wobei die aktive Okklusionsunterdrückung durch das Korrektursignal breitbandig erfolgt.

6. Hörsystem mit einem Hörinstrument (1), wobei das Hörinstrument (1) umfasst:
- einen ersten elektroakustischen Eingangswandler (10), welcher dazu eingerichtet ist, bei einem bestimmungsgemäßen Tragen des Hörinstruments (1) durch eine Testperson ein Schallsignal (22) in einem Gehörgang (4) der Testperson aufzunehmen, und daraus ein erstes Eingangssignal (24) zu erzeugen,
- einen elektroakustischen Ausgangswandler (18), welcher dazu eingerichtet ist einen Testschall (32) zu erzeugen und bei einem bestimmungsgemäßen Tragen des Hörinstruments (1) in den Gehörgang (4) der Testperson auszugeben,
wobei das Hörsystem (1) eine Steuereinrichtung (26) umfasst, welche dazu eingerichtet ist, eine Reaktion der Testperson auf den Testschall (32) zu registrieren und anhand besagter Reaktion der Testperson auf den Testschall (32) sowie mittels des ersten Eingangssignals (24) eine Hörschwelle der Testperson bei wenigstens einer Testfrequenz zu ermitteln, **dadurch gekennzeichnet, dass** das Hörinstrument (1) dazu eingerichtet ist,
- anhand des ersten Eingangssignals (24) ein Korrektursignal für eine aktive Okklusionsunterdrückung zu erzeugen, und durch den elektroakustischen Ausgangswandler (18) des Hörinstruments (1) anhand des Korrektursignals zur Kompensation eines Körperschalls (42) im Schallsignal (22) des Gehörgangs (4) ein Korrekturschall zu erzeugen und in den durch das Hörinstrument (1) wenigstens teilweise verschlossenen Gehörgang (4) der Testperson auszugeben, und/oder wobei das Hörinstrument zusätzlich einen zweiten elektroakustischen Eingangswandler (12) umfasst und das Hörinstrument dazu eingerichtet ist
- durch den zweiten elektroakustischen Eingangswandler (12) des Hörinstruments (1) einen Umgebungsschall (8) des Hörinstrumentes (1) aufzunehmen, und hieraus ein zweites Eingangssignal (28) zu erzeugen, und anhand des zweiten Eingangssignals (28) durch den elektroakustischen Ausgangswandler (18) des Hörinstruments (1) zusätzlich zur Ausgabe des Testschalls (32) eine aktive Rauschunterdrückung durchzuführen.

7. Hörsystem nach Anspruch 6, insofern das Hörinstrument einen zweiten elektroakustischen Eingangswandler (12) umfasst und dazu eingerichtet ist, einen Umgebungsschall (8) des Hörgerätes (2) aufzunehmen, und hieraus ein zweites Eingangssignal (28) zu erzeugen, wobei das Hörinstrument (1) als ein Hörgerät (2) ausgestaltet ist,
wobei die Steuereinrichtung (26) weiter dazu eingerichtet ist, anhand des zweiten Eingangssignals (28) ein Ausgangssignal zu erzeugen und dem elektroakustischen Ausgangswandler (18) zur Umwandlung in ein Ausgangsschallsignal zuzuführen.

8. Hörsystem nach Anspruch 7,
wobei die Steuereinrichtung (26) weiter dazu eingerichtet ist, anhand des zweiten Eingangssignals (28) mittels des elektroakustischen Ausgangswandlers (18) zusätzlich zur Ausgabe des Testschalls (32) eine aktive Rauschunterdrückung durchzuführen.

## Claims

1. A method for creating an audiogram of a subject by means of a hearing instrument (1),
wherein a sound signal (22) is recorded by a first electroacoustic input transducer (10) of the hearing instrument (1) in an auditory canal (4) of the subject, which is at least partially closed by the hearing instrument (1), and a first input signal (24) is generated therefrom, wherein a test sound (32) is generated by an electroacoustic output transducer (18) of the hearing instrument (1) and is output into the auditory canal (4) of the subject, which is at least partially closed by the hearing instrument (1), and wherein a hearing threshold of the subject at at least one test frequency is determined on the basis of a reaction of the subject to the test sound (32) and by means of the first input signal (24),
**characterized in that**
- a correction signal for active occlusion suppression is generated on the basis of the first input signal (24), and a correction sound is generated by the electroacoustic output transducer (18) of the hearing instrument (1) on the basis of the correction signal to compensate for a structure-borne sound (42) in the sound signal (22) of the auditory canal (4) and is output into the auditory canal (4) of the subject, which is at least partially closed by the hearing instrument (1), and/or
- ambient sound (8) of the hearing instrument (1) is recorded by a second electroacoustic input transducer (12) of the hearing instrument (1), and a second input signal (28) is generated therefrom, and active noise suppression is carried out on the basis of the second input signal (28) by the electroacoustic output transducer (18) of the hearing instrument (1) in addition to the output of the test sound (32).

2. The method as claimed in claim 1,
wherein the test sound (32) has a defined first signal contribution (34) at the at least one test frequency,
wherein, on the basis of the first input signal (24), a second signal contribution (40) of the sound signal (22) in the auditory canal (4) at the at least one test frequency is determined, and
wherein at least the second signal contribution (40) is taken into consideration for a determination of the hearing threshold in the at least one subject.

3. The method as claimed in claim 1 or claim 2,
wherein the correction sound is generated and output as a broadband sound signal, and
wherein a residual noise signal not purged by the active occlusion suppression is determined as the second signal contribution (40) on the basis of the first input signal (24).

4. The method as claimed in any one of the preceding claims, provided that active noise suppression is carried out,
wherein the active noise suppression is concentrated on a frequency range around the at least one test frequency.

5. The method as claimed in claim 4,
wherein the active noise suppression is concentrated on a frequency range around the at least one test frequency, and
wherein the active occlusion suppression by the correction signal is carried out in a broadband manner.

6. A hearing system having a hearing instrument (1), wherein the hearing instrument (1) comprises:
- a first electroacoustic input transducer (10), which is configured, when the hearing instrument (1) is worn as intended by subject, to record a sound signal (22) in an auditory canal (4) of the subject, and to generate a first input signal (24) therefrom,
- an electroacoustic output transducer (18), which is configured to generate a test sound (32), and, when the hearing instrument (1) is worn as intended, to output it into the auditory canal (4) of the subject,
wherein the hearing system (1) comprises a control device (26), which is configured to register a reaction of the subject to the test sound (32) and to determine a hearing threshold of the subject at at least one test frequency on the basis of said reaction of the subject to the test sound (32) and by means of the first input signal (24),
**characterized in that**
the hearing instrument (1) is configured
- to generate a correction signal for active occlusion suppression on the basis of the first input signal (24), and to generate a correction sound by the electroacoustic output transducer (18) of the hearing instrument (1) on the basis of the correction signal to compensate for a structure-borne sound (42) in the sound signal (22) of the auditory canal (4) and to output it into the auditory canal (4) of the subject, which is at least partially closed by the hearing instrument (1), and/or wherein the hearing instrument additionally comprises a second electroacoustic input transducer (12) and the hearing instrument is configured
- to record ambient sound (8) of the hearing instrument (1) by way of the second electroacoustic input transducer (12) of the hearing instrument (1), and to generate a second input signal (28) therefrom, and to carry out active noise suppression on the basis of the second input signal (28) by the electroacoustic output transducer (18) of the hearing instrument (1) in addition to the output of the test sound (32).

7. The hearing system as claimed in claim 6, provided that the hearing instrument comprises a second electroacoustic input transducer (12) and is configured to record ambient sound (8) of the hearing aid (2) and to generate a second input signal (28) therefrom,
wherein the hearing instrument (1) is designed as a hearing aid (2),
wherein the control unit (26) is furthermore configured to generate an output signal on the basis of the second input signal (28) and to supply it to the electroacoustic output transducer (18) for conversion into an output sound signal.

8. The hearing system as claimed in claim 7,
wherein the control device (26) is furthermore configured to carry out active noise suppression on the basis of the second input signal (28) by means of the electroacoustic output transducer (18) in addition to the output of the test sound (32).

## Revendications

1. Procédé permettant de créer un audiogramme d'une personne testée au moyen d'un appareil auditif (1), dans lequel un premier transducteur d'entrée électroacoustique (10) de l'appareil auditif (1) enregistre un signal sonore (22) dans un conduit auditif (4), au moins partiellement fermé par l'appareil auditif (1), de la personne testée, et génère un premier signal d'entrée (24) à partir de celui-ci,
dans lequel un transducteur de sortie électroacoustique (18) de l'appareil auditif (1) génère un son test (32) et l'émet dans le conduit auditif (4), au moins partiellement fermé par l'appareil auditif (1), de la personne testée, et dans lequel
un seuil auditif de la personne testée à au moins une fréquence test est déterminé à l'aide d'une réaction de la personne testée au son test (32) et au moyen du premier signal d'entrée (24),
**caractérisé en ce que**
- un signal de correction est généré à l'aide du premier signal d'entrée (24) pour une suppression d'occlusion active, et le transducteur de sortie électroacoustique (18) de l'appareil auditif (1) génère à l'aide du signal de correction un son de correction pour compenser un bruit solidien (42) dans le signal sonore (22) du conduit auditif (4) et l'émet dans le conduit auditif (4), au moins partiellement fermé par l'appareil auditif (1), de la personne testée, et/ou
- un deuxième transducteur d'entrée électroacoustique (12) de l'appareil auditif (1) enregistre un son ambiant (8) de l'appareil auditif (1), et génère un deuxième signal d'entrée (28) à partir de celui-ci, et à l'aide du deuxième signal d'entrée (28), le transducteur de sortie électroacoustique (18) de l'appareil auditif (1) exécute de plus une suppression de bruit active pour émettre le son test (32).

2. Procédé selon la revendication 1,
dans lequel le son test (32) présente une première contribution de signal (34) définie à ladite au moins une fréquence test,
dans lequel à l'aide du premier signal d'entrée (24), une deuxième contribution de signal (40) du signal sonore (22) dans le conduit auditif (4) est déterminée à ladite au moins une fréquence test, et
dans lequel au moins la deuxième contribution de signal (40) est prise en compte pour la détermination du seuil auditif chez ladite au moins une personne testée.

3. Procédé selon la revendication 1 ou la revendication 2,
dans lequel le son de correction est généré et émis sous la forme d'un signal sonore à large bande, et
dans lequel un signal de bruit résiduel, non rectifié par la suppression d'occlusion active, est déterminé comme la deuxième contribution de signal (40) à l'aide du premier signal d'entrée (24).

4. Procédé selon l'une quelconque des revendications précédentes, dans la mesure où une suppression de bruit active est effectuée,
dans lequel la suppression de bruit active se concentre sur une plage de fréquences autour de ladite au moins une fréquence test.

5. Procédé selon la revendication 4,
dans lequel la suppression de bruit active se concentre sur une plage de fréquences autour de ladite au moins une fréquence test, et
dans lequel la suppression d'occlusion active est effectuée à large bande par le signal de correction.

6. Système auditif, comprenant un appareil auditif (1), l'appareil auditif (1) comprenant :
- un premier transducteur d'entrée électroacoustique (10) qui est conçu pour enregistrer un signal sonore (22) dans un conduit auditif (4) de la personne testée lorsqu'une personne testée porte correctement l'appareil auditif (1) et pour générer un premier signal d'entrée (24) à partir de celui-ci,
- un transducteur de sortie électroacoustique (18) qui est conçu pour générer un son test (32) et pour l'émettre dans le conduit auditif (4) de la personne testée lorsque l'appareil auditif (1) est porté correctement,
dans lequel le système auditif (1) comprend un dispositif de commande (26) qui est conçu pour consigner une réaction de la personne testée au son test (32) et pour déterminer à l'aide de ladite réaction de la personne testée au son test (32) ainsi qu'au moyen du premier signal d'entrée (24) un seuil auditif de la personne testée à au moins une fréquence test, **caractérisé en ce que**
l'appareil auditif (1) est conçu
- pour générer à l'aide du premier signal d'entrée (24), un signal de correction pour une suppression d'occlusion active, et pour générer avec le transducteur de sortie électroacoustique (18) de l'appareil auditif (1) un son de correction à l'aide du signal de correction afin de compenser un bruit solidien (42) dans le signal sonore (22) du conduit auditif (4) et pour l'émettre dans le conduit auditif (4), au moins partiellement fermé par l'appareil auditif (1), de la personne testée, et/ou dans lequel l'appareil auditif comprend de plus un deuxième transducteur d'entrée électroacoustique (12), et l'appareil auditif est conçu
- pour enregistrer par le deuxième transducteur d'entrée électroacoustique (12) de l'appareil auditif (1) un son ambiant (8) de l'appareil auditif (1), et pour générer un deuxième signal d'entrée (28) à partir de celui-ci, et pour effectuer de plus une suppression de bruit active pour émettre le son test (32) à l'aide du deuxième signal d'entrée (28) par le transducteur de sortie électroacoustique (18) de l'appareil auditif (1).

7. Système auditif selon la revendication 6, dans la mesure où l'appareil auditif comprend un deuxième transducteur d'entrée électroacoustique (12) et est conçu pour enregistrer un son ambiant (8) de l'appareil auditif (2) et pour générer un deuxième signal d'entrée (28) à partir de celui-ci, l'appareil auditif (1) étant configuré sous forme d'aide auditive (2),
dans lequel le dispositif de commande (26) est en outre conçu pour générer à l'aide du deuxième signal d'entrée (28) un signal de sortie et pour l'acheminer au transducteur de sortie électroacoustique (18) afin de le convertir en signal sonore de sortie.

8. Système auditif selon la revendication 7,
dans lequel le dispositif de commande (26) est en outre conçu pour effectuer de plus une suppression de bruit active pour l'émission du son test (32), à l'aide du deuxième signal d'entrée (28) au moyen du transducteur de sortie électroacoustique (18).
